# EUROPEAN PATENT APPLICATION

(11) **EP 3 707 993 A1**
(43) Date of publication of application: **16.09.2020**
(21) Application number: 19162136.6
(22) Date of filing: 12.03.2019
(51) Int. Cl.: A01H 6/20, A01H 4/00, C12N 5/04

(54) **PRODUCING ISOTHIOCYANATES FROM CALLUS SUSPENSION CULTURES**

(71) Applicant: Perpetuum CropScience BVBA, 2610 Antwerpen (BE)
(72) Inventor: HOFENK, Jeroen, 2000 Antwerpen (BE); TROCH, James, 2610 Antwerpen (BE)
(74) Representative: Clerinx, Peter Albert I.

(57) **Abstract**

The present invention provides a method of producing isothiocyanates, The method comprises forming, in a semisolid or solid callus induction medium, compact callus aggregates from cells obtained from explant material of a plant of a Brassicales plant species. The method comprises transferring cells from the callus aggregates, e.g. transferring the callus aggregates, to a suspension culture in a liquid medium in a shake flask, the liquid medium containing at least one elicitor and/or adsorbent. The method comprises transferring, after culturing in the shake flask, cells from the suspension culture to a further suspension culture in a bioreactor containing the at least one elicitor and/or adsorbent. The method comprises extracting and/or purifying of at least one isothiocyanate from cells obtained from the bioreactor.

## Description

### Technical field

The present invention relates to the field of in vitro production of isothiocyanates, i.e. using a plant cell culture. More specifically, the invention relates to a method of producing isothiocyanates from cell material of a Brassicales, and applications thereof.

### Technical background

Isothiocyanates, having a r-N = C = s structure as general formula, are important functional substances in natural products and pharmaceutically active compounds. These compounds are important organic synthesis intermediates, and have wide applications within pharmaceutical, nutraceutical, cosmetics and agricultural industries. Isothiocyanates can participate in a variety of organic reachtions, and are used for synthesizing various types of compounds containing sulfur, nitrogen and oxygen, especially heterocyclic compounds. Isothiocyanates can be widely applied to prepare organic synthesis products, such as medicines, cosmetic raw materials, pesticides, dyes and the like. Isothiocyanates can also be used for determining the amino acid sequence in peptides and proteins and serving as a fluorescein marker.

Isothiocyanates can be produced by several plants belonging to Brassicales (or Cruciales) plant species. Brassicales are an order of flowering plants, belonging to the Eurosids II group of dicotyledons under the APG II system. One character common to many members of the order is the production of glucosinolate (mustard oil) compounds. Most systems of classification have included this order, although sometimes under the name Capparales (the name chosen depending on which is thought to have priority). Particular popular vegetable and medicinal foods in these classifications include among others; broccoli, Brussels sprouts, cabbage, cauliflower, wasabi, horseradish, turnip, kale, mustard species, watercress, papaya seeds, moringa seeds, nasturtiums, and capers. These species generate isothiocyanates in different proportions, and so have different but recognizably related flavors. Isothiocyanates function as a system of defense against pathogen attack. Many of the aforementioned plants can be classified as medicinal plants and are also suitable for consumption. They contain a wide range of phytochemicals, such as alkaloids, flavonoids, phenols, and glucosinolates and are considered as an important source of life saving drugs for the majority of the world's population. Among these secondary metabolites, glucosinolates and especially their hydrolytic products, isothiocyanates, have unparalleled bioactivity compared to the different plant phytochemicals. Isothiocyanate compounds have the potential to act as an anti-insecticide, antioxidant, herbicide, antitumorigenic, and anticancer compound.

Isothiocyanates are the aliphatic and aromatic compounds arising from the hydrolysis (breakdown) of glucosinolates by the endogenous enzyme myrosinase (β-thioglucosidase). Myrosinase , an enzyme that catalyzes the hydrolysis of glucosinolates, is stored in myrosinase grains in the vacuoles of certain phloem cells in the main types of plant tissue: ground tissues, vascular tissues, and epidermis. These cells are therefore called myrosin cells. The enzyme myrosinase is thus physically separated from glucosinolates in the intact plant cells. When the aforementioned plants are chopped or chewed, myrosinase can interact with glucosinolates and release isothiocyanates from their precursors.

The aforementioned plants all contain a variety of glucosinolates, each of which forms a different isothiocyanate when hydrolyzed. For example, broccoli is a good source of glucoraphanin, the glucosinolate precursor of sulforaphane, and sinigrin, the glucosinolate precursor of allyl isothiocyanate. Watercress is a rich source of gluconasturtiin, the precursor of phenethyl isothiocyanate, while garden cress is rich in glucotropaeolin, the precursor of benzyl isothiocyanate.

Once absorbed, glucosinolate-derived isothiocyanates are promptly conjugated to glutathione by a class of phase II detoxification enzymes known as glutathione S-transferases (GSTs) in the liver, and then sequentially metabolized in the mercapturic acid pathway. This mechanism is meant to increase the solubility of isothiocyanates, thereby promoting a rapid excretion in the urine.

Presently, a research interest exists in the cancer-preventive activities of vegetables that are rich in glucosinolates and of the individual isothiocyanates.

Indeed, numerous studies confirm that isothiocyanates are approximately six times more bioavailable than glucosinolates, which are biologically inactive and must first be hydrolyzed in order to be activated. Therefore, isothiocyanates have been well sought after as subjects of research for more than half a century. Interest in these unique secondary metabolites escalated following the discovery that sulforaphane, an isothiocyanate from broccoli, potently induces mammalian cytoprotective proteins through the Keap1-Nrf2-ARE pathway.

Furthermore, considerable research and development advances have also been achieved with benzylisothiocyanate, generally classified as one of the chemo-preventive agents that are thought to prevent carcinogenic and other genotoxic compounds from reaching or reacting with the target sites on the treated tissue. Higher intakes of benzylisothiocyanate correlate with reduced risk of cancers of the lung, breast, and colon while blocking cancer development. Among the various forms of isothiocyanates, benzylisothiocyanate exhibits one of the strongest anticancer effects. Studies have shown that it helps to prevent lung cancer and esophageal cancer, and can also lower the risk of other cancers, including gastrointestinal cancer. Benzyl Isothiocyanate efficiently inhibits several cancer promoting-cytochrome enzymes, helping to prevent carcinogenesis. It has also been reported that Benzyl isothiocyanate induces apoptosis in human pancreatic cancer cells and inhibits growth of cultured and xenografted human breast cancer cells.

In parallel with the advances in understanding the molecular regulations of isothiocyanates and their critical role in protection against electrophiles and oxidants, there have been increased efforts toward translating this knowledge to improve human health and combat disease. Considerable attention from the industry therefore, goes into achieving new production protocols that are cost-effective, produce high yields, while ensuring working under green chemistry principles, without exploiting natural plant resources.

When preparing isothiocyanate compounds, especially high purity grades, the preparation method needs to conform to the medicinal standards, and the development of the preparation method has to be capable of realizing industrial production while being environmentally friendly and cost-effective. At present, the methods of synthesis for isothiocyanates are numerous: a phosgene method, a sulfur-phosgene method, a carbon disulfide method and a bis (trichloromethyl) method, a carbonate method, a thiourea decomposition method, a phenyl thiochloroformate method, a thiocyanate method and the like.

In the phosgene route, phenylethylamine and carbon disulfide are dissolved in an organic solvent, introducing phosgene in the presence of alkali to generate phenethyl isothiocyanate. The phosgene can also be used in the form of an alternative product of phosgene, such as ethyl chloroformate, double phosgene, triphosgene and the like. However, the reaction process still cannot avoid the generation of phosgene, which can pollute the environment and has a relatively large potential safety hazard. The method of producing isothiocyanate by direct reaction of thiophosgene and amine compound has a wide application range, is rapid in reaction and capable of replacing phosgene with sulfur phosgene, so that the safety is improved. However, the sulfur phosgene is a volatile liquid with toxicity, and the environmental hazard is substantially large, the production and transportation are not safe, and the route is not beneficial to large-scale industrial production;

In the isocyanide route, isothiocyanate is synthesized from isocyanide and sulfur powder or vulcanizing agent in the presence of a metal catalyst. The main problem of this approach is that the synthesis and purification difficulty of isocyanide is very high. Furthermore, isocyanide is a highly toxic substance and therefore cannot be used for production with consumption in mind.

In the thiocyanate route, reaction of halogenated hydrocarbon and thiocyanate is used to produce isothiocyanate. However, this method has the disadvantage that the overall yield is relatively low, and the process is quite tedious.

In the carbon disulfide route, a production method can comprise the following steps: dissolving phenylethylamine (or other amine organic compounds) and carbon disulfide in an organic solvent, and synthesizing the amido dithiocarbamate under the catalysis of alkali, and reacting under the action of a desulfurizing agent to obtain the isothiocyanate compound. With regard to selection of a desulfurizing agent, much research has to be carried out, and the desulfurizing agent is mainly composed of methyl chloroformate and p-toluene sulfonyl chloride, solid phosgene, elemental iodine, chlorosilane, chlorine phosphate or dicyclohexylcarbodiimide and the like. The method has the advantage that highly toxic raw materials are avoided, but the purity of the obtained product is low. A following purification protocol can be carried out by column chromatography. The overall operation is tedious, and the reagent dosages are large, making this method more suitable for experimental grade reaction, and less suitable for industrial production.

In view of this, a need exists in the art to develop a safe, simple, environment-friendly and cost-effective process, capable of realizing the preparation and purification methods of isothiocyanate compounds for industrial production, resulting in high-purity and high-quality isothiocyanate compounds that meet the ever-increasing medicinal and industrial production requirements with great respect of green chemistry principles and most importantly, without exploiting natural plant resources.

### Summary

It is an object of embodiments of the present invention to provide efficient, reproducible, safe, simple, environment-friendly, and/or cost-effective means and methods for, or relating to, the production of isothiocyanate.

It is an advantage of embodiments of the present invention that means and methods are provided for producing isothiocyanates from biological material on a constant and industrial scale.

It is an advantage of embodiments of the present invention that a cost-effective, environmentally friendly, and/or more efficient alternative over traditional field cultivation and chemical synthesis on industrial scale of commercially valuable isothiocyanates is provided.

It is an advantage of embodiments of the present invention that isothiocyanates can be produced without, or with very limited, interference from interactions between environmental and seasonal factors with the plant.

It is an advantage of embodiments of the present invention that a higher production effectiveness can be achieved in producing isothiocyanates from callus cultures of Brassicales (Cruciales) plants, including, without limitation, the following plant families: Akaniaceae, Bataceae, Brassicaceae, Capparaceae, Caricaceae, Cleomaceae, Gyrostemonaceae, Koeberlineaceae, Limnanthaceae, Moringaceae, Pentadiplandraceae, Resedaceae, Salvardoraceae, Setchellanthaceae, Tovariaceae, and Tropaeolaceae.

It is an advantage of embodiments of the present invention that one or more disadvantages of a prior art method for producing isothiocyanates, such as methods and their associated disadvantages that were briefly outlined hereinabove, may be overcome or alleviated.

Other objects, advantages, and novel features will be readily apparent to those skilled in the art from the following detailed description of the invention.

In a first aspect, the present invention provides a method of producing isothiocyanates, The method comprises forming, in a semi-solid or solid callus induction medium, compact callus aggregates from cells obtained from explant material of a plant of a Brassicales plant species. The method may comprise obtaining the cells from the explant material and/or obtaining the explant material. The method comprises transferring cells from the callus aggregates, e.g. transferring the callus aggregates, to a suspension culture in a liquid medium in a shake flask, the liquid medium containing at least one elicitor and/or adsorbent. The method comprises transferring, after culturing in the shake flask, cells from the suspension culture to a further suspension culture in a bioreactor containing the at least one elicitor and/or adsorbent. The method comprises extracting and/or purifying of at least one isothiocyanate from cells obtained from the bioreactor.

A method in accordance with embodiments of the present invention may comprise initiating compact callus aggregates of leaf in the semi-solid or solid induction medium and sub-culturing and expansion of the compact callus aggregates in a further semi-solid or solid callus induction medium, e.g. a fresh semi-solid or solid induction medium, e.g. having a same or similar composition as the semi-solid or solid induction medium, for example after about 4 weeks of initiating the compact callus aggregates in the semi-solid or solid induction medium.

In a method in accordance with embodiments of the present invention, extracting and/or purifying may comprise isolating the at least one isothiocyanate as a secondary metabolite of the cells from the bioreactor by solvent extraction.

In a method in accordance with embodiments of the present invention, the cells obtained from explant material may be are derived from leaves, fruit, shoots, buds, flowers, bark, roots, branches, stems, seeds, cones, needles or cambium tissue of the plant.

In a method in accordance with embodiments of the present invention, the cells obtained from explant material may be obtained from hypocotyl explant material and/or wherein said cells are derived from meristematic plant tissue.

In a method in accordance with embodiments of the present invention, the cells obtained from explant material may be obtained from explant material of a plant species that produces the secondary metabolite benzyl isothiocyanate and/or other isothiocyanates.

In a method in accordance with embodiments of the present invention, the cells obtained from explant material may be Brassica oleracea L plant cells.

In a method in accordance with embodiments of the present invention, the callus induction medium may comprise a Murashige and Skoog medium and/or a B-5 medium,

In a method in accordance with embodiments of the present invention, the at least one elicitor may comprise at least one abiotic elecitor and/or at least one biotic elicitor and/or at least one microbial fraction or product derived from a biotic elicitor.

In a method in accordance with embodiments of the present invention, the at least one elicitor may comprise chitosan and/or salicylic acid to increase accumulation of benzyl isothiocyanate or any other isothiocyanate.

In a method in accordance with embodiments of the present invention, the at least one adsorbent may comprise an aliphatic adsorbent and/or an immisible liquid phase adsorbent.

In a method in accordance with embodiments of the present invention, the at least one elicitor and/or adsorbent may be added to the suspension culture, and/or to the further suspension culture, at a time from an early exponential growth phase to a stationary phase of the culture.

In a method in accordance with embodiments of the present invention, the at least one elicitor and/or adsorbent may be added at a plurality of times to the suspension culture and/or to the further suspension culture, in which said plurality of times are separated by a period in the range of about six hours to about a month.

In a method in accordance with embodiments of the present invention, the induction medium and/or the liquid medium may comprise one or more of: a carbon source, an organic nitrogen source, an inorganic nitrogen source, a macrosalt, a microsalt, a rare trace element, a vitamin, an organic supplement, a plant hormone, a hormone substitute or derivative, a hormone inhibitor, a synthetic growth regulator, a biosynthetic precursor, a metabolic inhibitor, a non-metabolic inhibitor, a stimulant, an activator, an anti-browning agent, an anti-oxidant, a stabiliser, an enhancer, a radical, a scavenger, a conditioner and a reducing agent.

In a method in accordance with embodiments of the present invention, the induction medium may comprise an auxin and/or a cytokinin and/or a giberellin.

In a method in accordance with embodiments of the present invention, the callus aggregates and/or the suspension culture and/or the further suspension culture may be repeatedly sub-cultured (e.g. on a weekly, fortnightly or monthly basis). Preferably, the compact callus aggregates may be transferred after about 4 weeks to a fresh culture medium, e.g. having a same or similar composition as the callus induction medium.

In a method in accordance with embodiments of the present invention, the step initiating, transferring toa shake flask and/or transferring to a bioreactor may be conducted as a batch process and/or in a semi-continuous process and/or in a continuous process. The semi-continuous process may be operated in a fed-batch or a repeated-batch mode. In a method in accordance with embodiments of the present invention, the sub-culturing may be conducted weekly, fortnightly or monthly. The semi-continuous process may be operated in a fed-batch or a repeated-batch mode in which the cultivation duration may be between about two days to about several months, preferably between about six days and about twenty days in duration. The continuous process can be operated in a two-phase system in which the plant cells may be growing in a bioreactor system in either suspension or immobilization, and the medium is circulated between the bioreactor and an absorbant reservoir or resin column for secondary metabolite adsorption. Further iterations, or continuous application of a method in accordance with embodiments of the present invntion may start from cells derived from regularly sub-cultured callus culture and/or suspension cell culture, e.g. as obtained in a previous iteration or during previous application of the method..

In a method in accordance with embodiments of the present invention, the cells or those from which they are derived may have been subjected to genetic manipulation.

In a second aspect, the present invention relates to a use of a method in accordance with embodiments of the present invention for manufacturing a pharmaceutical product, a food or drink product, a supplement, an additive, a skin care product, a hair care product, or an agricultural product.

The independent and dependent claims describe specific and preferred features of the invention. Features of the dependent claims can be combined with features of the independent claims and with features of other dependent claims as deemed appropriate, and not necessarily only as explicitly stated in the claims.

### Short description of the drawings

FIG 1 shows a comparison of benzyl isothiocyanate content as function of time for cell suspension cultures in respectively an Erlenmeyer flask and in a bioreactor, in an example illustrating embodiments of the present invention.
FIG 2 shows a callus of NON-GMO Impala F1 Cabbage (Brassica Oleracea L. var. Capitata), in an example illustrating embodiments of the present invention.

The drawings are schematic and not limiting. Elements in the drawings are not necessarily represented on scale. The present invention is not necessarily limited to the specific embodiments of the present invention as shown in the drawings.

### Detailed description

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

The embodiments of the present invention are specifically described below with reference to the embodiments, so as to facilitate the understanding of the present invention by those skilled in the art. It should be noted that the embodiments are only used for further explanation of the present invention. The embodiments cannot be understood to limit the protection scope of the present invention. A person skilled in the art will recognize that the protection scope of the present invention can be better understood by those skilled in the art. The non-essential improvement and adjustment made by the method disclosed by the invention should still be within the protection scope of the invention. Meanwhile, raw materials being used are not always described in detail. The raw materials may be commercially available products. The process steps or preparation methods that are not always described in detail are process steps or preparation methods which are known by those skilled in the art.

Since many plant sources of high-value metabolites are not domesticated, these plant species often grow slow, their population is limited, and the concentration of the target molecule is highly variable and routinely present at extremely low concentrations, thus complicating large-scale extraction. The total chemical synthesis of metabolites can provide an attractive route for production of simple molecules. However, many metabolites have multiple chiral centres with region-specific and stereo-specific properties associated to their function, making total chemical synthesis either difficult or unprofitable. The production of metabolites via semi-synthesis represents another approach that can circumvent some of the issues associated with the total synthesis of these high-value plant chemicals, even though semi-synthesis routes can remain costly and often generate toxic by-products, which can be damaging to the environment. The use of plant cell cultures for the synthesis of metabolites provides several advantages in comparison to other potential strategies, especially for the production of metabolites with complex structures. For example, secondary metabolites can be produced without exploiting natural plant resources. The rate of cell growth and biosynthesis in callus culture initiated from a small amount of plant material can be quite high, and can be indefinitely maintained through regular sub-culturing. Production of plant secondary products by in-vitro growing of large amounts of undifferentiated tissues can circumvent seasonality and time specificity of plant production.

Callus is, without limitation, an unorganized tissue mass growing on solid substrate. Callus forms naturally on plants in response to wounding or infestations and at graft. Callus can be multiplied indefinitely through regular sub-culturing and can be used to clone numerous whole plants. Callus formation can be induced by elevated hormone levels. For example, tissue culture media for producing callus may contain cytokinins and auxins as additive. During callus formation, some degree of dedifferentiation can occur both in morphology (a callus is usually composed of unspecialized parenchyma cells) and metabolism, e.g. changes during development and specialization are, to some extent, reversed.

One consequence of this dedifferentiation is that most plant cultures lose the ability to photosynthesize. This has important consequences for the culture of callus tissue, as the metabolic profile will probably not match that of the donor plant. This may prompt the addition of other components such as vitamins and, most importantly, a carbon source to the culture medium, in addition to the usual mineral nutrients. Callus culture is often performed in the dark (the lack of photosynthetic capability being no drawback), since light can encourage differentiation of the callus. During long term culture, the culture may lose the requirement for auxin and/or cytokinin. This process, known as habituation, is common in callus cultures from some plant species (such as sugar beet). Nonetheless, callus cultures are extremely important in plant biotechnology and to further extend in pharmaceuticals, nutraceuticals, cosmetics, agriculture as well.

The inventors' ongoing research on isothiocyanates, its health benefits and extraction methods that emphasize the importance of green chemistry resulted in achieving a method of extracting isothiocyanates from callus culture. A method is disclosed hereinbelow that is in line with the current economic and industry trends and offers a suitable cost-effective, environmentally friendly, and more efficient alternative over traditional field cultivation and/or chemical synthesis on industrial scale.

In a first aspect, the present invention relates to a method for producing isothiocyanates. The method comprises forming, in a semi-solid or solid callus induction medium, compact callus aggregates from cells obtained from explant material of a plant of a Brassicales plant species and transferring cells from the callus aggregates, e.g. transferring the callus aggregates, to a suspension culture in a liquid medium in a shake flask for culturing. The liquid medium contains at least one elicitor and/or adsorbent. The method comprises transferring the cells from the shake flask to a further suspension culture in a bioreactor, e.g. a large wave bioreactor, containing the at least one elicitor and/or adsorbent, and extracting and/or purifying of at least one isothiocyanate from cells obtained from the bioreactor.

The method in accordance with embodiments of the present invention is a method for producing isothiocyanates, such as benzyl isothiocyanate, sulforaphane, sulforaphene, raphanin, allyl isothiocyanate, methyl isothiocyanate, fluorescein isothiocyanate, erysolin, erucin, iberin, alyssin, berteroin, iberverin, cheirolin, 5-methylsulfinylpentyl isothiocyanate, 6-methylsulfinylhexyl isothiocyanate, 7-methylsulfinylheptyl isothiocyanate, 8-methylsulfinyloctyl isothiocyanate, phenylethyl isothiocyanate, 4-(α.-L-rhamnopyranosyloxy)benzyl isothiocyanate, 3-(α.-L-rhamnopyranosyloxy)benzyl isothiocyanate, 2-(α L-rhamnopyranosyloxy)benzyl isothiocyanate, 4-(4'-O-acetyl-.alpha.-L-rhamnopyranosyloxy)benzyl isothiocyanate, and/or any derivative thereof.

The method comprises forming, e.g. initiating, in a semi-solid or solid callus induction medium, compact callus aggregates from cells obtained from explant material of a plant of a Brassicales plant species, e.g. glucosinolate-containing plant tissue material.

The method may comprise surface-sterilizing the explant material prior to introduction into the callus induction medium. Conventional sterilisation techniques such as the use of 70% ethanol, 15% sodium hypochlorite and "Chlorox" (a bleach commercially available from the Chlorox company) treatment may be used. In addition, antimicrobial agents such as cefoxitin, benlate, cloxacillin, ampicillin, gentamycin sulphate and phosphomycin may be used for surface sterilisation of plant material. The explants taken from the plant may thus be utilised to establish a callus culture.

The cells may be derived from leaves, fruit, shoots, buds, flowers, bark, roots, branches, stems, seeds, cones, needles or cambium tissue of the plant. The cells may be obtained from hypocotyl explant material. The cells may be derived from meristematic plant tissue. In a particularly preferred embodiment of the invention, the cells may be derived from hypocotyl meristematic end plant tissue. In a preferred embodiment, the cells are obtained from explant material of a plant species that produces the secondary metabolite benzyl isothiocyanate and/or other isothiocyanates. For example, the cells may be Brassica oleracea L. plant cells, e.g. var. Capitata. The cells may be cells of NON-GMO Impala F1 Cabbage (Brassica oleracea L. var. Capitata).

Due to the heterogeneous population of typical dedifferentiated cells that comprise callus, the selection of a highly productive cell line may be considered an important, and non-obvious, step for the establishment of profitable production platforms for metabolites. As the accumulation of metabolites in plants is genotype specific, the selection of suitable species and subsequently organs for callus generation may be important. In addition, this selection process can be facilitated by elegant chemical-based approaches. For example, the identification of cell lines exhibiting a high level of metabolic flux through the targeted pathway by the exogenous application of an intermediate. As per exemplification of the present invention, without limitation, callus aggregates of hypocotyls explants of NON-GMO Impala F1 Cabbage (Brassica oleracea L. var. Capitata) can be used for the production and extraction of benzyl isothiocyanate.

The callus induction medium may comprise sucrose, e.g. 3% by mass of analytical grade sucrose. The callus induction medium may comprise a gelling agent, e.g. 0.6% by mass of Phytagel (CAS Number 71010-52-1 , available from Sigma-Aldrich Co. LLC).

The callus induction medium may comprise a Murashige and Skoog medium and/or a Gamborg B-5 medium, The callus induction medium may comprise one or more of: a carbon source, an organic nitrogen source, an inorganic nitrogen source, a macrosalt, a microsalt, a rare trace element, a vitamin, an organic supplement, a plant hormone, a hormone substitute or derivative, a hormone inhibitor, a synthetic growth regulator, a biosynthetic precursor, a metabolic inhibitor, a non-metabolic inhibitor, a stimulant, an activator, an anti-browning agent, an anti-oxidant, a stabiliser, an enhancer, a radical, a scavenger, a conditioner and a reducing agent.

The callus induction medium may be a basic culture medium. Before use, the medium may be subjected to highpressure sterilization treatment, e.g. at 121°C for 25 minutes.

The callus induction medium may comprise an auxin, e.g. 2,4-dichlorophenoxyacetic acid (2,4-d) and/or a cytokinin, e.g. 6-benzylaminopurine (BAP), and/or a giberellin. For example, the medium may comprise 2.4-d, e.g. at a concentration of 3.0mg/l, and BAP, e.g. at a concentration of 1.0mg/l. It was observed that the latter combination has a remarkable induction effect on Brassica calli, e.g. on organic NON-GMO Impala F1 Cabbage (Brassica oleracea L. var. Capitata) calli. Other ratios of growth regulators as well as the use of different auxins, cytokinins and/or giberellins are also within the scope of present invention.

Preferably, the compact callus aggregates may be transferred after about 4 weeks to a fresh culture medium, e.g. having a same or similar composition as the callus induction medium to sub-culture and expand the compact callus aggregates.

The method comprises transferring the calli (or cells of the calli) to a medium scale suspension culture in liquid medium in at least one shake flask and culturing the suspension culture in the liquid medium. Following successful callus formation, cell suspension cultures are generated, simply by adding cells obtained from the calli, e.g. the calli or cells obtained therefrom, to a liquid medium, with the addition of elicitors and/or absorbents to ramp up secondary metabolite production. The resulting suspension culture, after establishment of the medium scale suspension culture, has significant scale-up capability for their growth within industrially relevant bioreactors designed to maximize levels of metabolite biosynthesis. "Suspension culture" is used to describe structurally undifferentiated cells that are dispersed in a liquid nutrient medium. It is understood that suspension cultures can comprise cells in various stages of aggregation. For example, a range of aggregate sizes can be encountered in a suspension in accordance with embodiments of the present invention, with sizes ranging from tens of microns in diameter (single cells or few-aggregated cells) to aggregates many millimetres in diameter, consisting of many thousands of cells.

Callus cultures will typically exhibit variability in growth morphology, productivity, product profiles and other characteristics. Since individual cell lines vary in their preferences for growth medium constituents, many different growth media may be used for induction and proliferation of the callus, e.g. in a callus induction medium and/or a liquid medium used in a method in accordance with embodiments of the present invention. The appropriate medium composition may vary depending upon the species being cultured.

Plant suspension cultures are capable of rapid growth rates and high cell densities. However, optimal conditions vary from one cell line to another, and accordingly, methods leading towards rapid optimisation for any given cell line must be considered. The initial cultures of various species may be sub-cultured by transfer into suitable suspension culture nutrient medium.

The liquid medium may comprise one or more of: a carbon source, an organic nitrogen source, an inorganic nitrogen source, a macrosalt, a microsalt, a rare trace element, a vitamin, an organic supplement, a plant hormone, a hormone substitute or derivative, a hormone inhibitor, a synthetic growth regulator, a biosynthetic precursor, a metabolic inhibitor, a non-metabolic inhibitor, a stimulant, an activator, an anti-browning agent, an anti-oxidant, a stabiliser, an enhancer, a radical, a scavenger, a conditioner and a reducing agent.

Certain classes of additives in the nutrient medium are referred to by special names in this invention, and are defined here. As used herein, the term "anti-browning agents" refers to components that are added to the medium to prevent the formation of pigments during cell cultivation. These pigments include phenolics and related compounds that are generally observed to have a deleterious effect on cell growth, viability, and production formation. As used herein, the term "biosynthetic precursors" is used to describe compounds added to the nutrient medium that are metabolised and incorporated by the cells into the metabolites of interest (for example Benzyl Isothiocyanate). As used herein, the term "metabolic inhibitors" is used to describe compounds added to the nutrient medium that interfere with specific biosynthetic pathways. For example, a metabolic inhibitor may be used to enhance biosynthesis by blocking a different pathway that competes with secondary metabolite for an early biosynthetic precursor. As used herein, the term stimulator or activator is used to describe compounds added to the nutrient medium that stimulate or activate specific biosynthetic pathways, for example those leading to biosynthesis. It is understood that the mechanism of action of the additives described herein may not be completely understood.

It is understood that modifications may be made in the medium, insofar allowed for in embodiments of the present invention as constrained by the scope of the claims, such as substitution of other conventional salt compositions (such as organics, vitamins, amino acids, precursors, activators and inhibitors), addition or deletion of various components, growth regulators or alteration of proportions.

In addition to non-volatile dissolved nutrients, gaseous components, primarily oxygen, carbon dioxide, and ethylene (a plant hormone), play critical roles in growth and product formulation. Two parameters are important. The dissolved gas concentrations favouring growth and secondary metabolite formation are obviously important since they dictate reactor operating conditions. In addition, the rates of consumption or production need to be incorporated into reactor design, so that the optimum specified concentrations can be maintained.

The liquid medium comprises at least one elicitor and/or adsorbent. The elicitor and/or adsorbent may be present in the liquid medium before adding the calli, or after adding the calli, e.g. at one or more predetermined times.

The at least one elicitor may comprise at least one abiotic elecitor and/or at least one biotic elicitor and/or at least one microbial fraction or product derived from a biotic elicitor. The yield of the secondary metabolite produced in the suspension cell culture in a method in accordance with embodiments can be increased by including within the suspension culture the one or more elicitors. As used herein, the term "elicitor" encompasses compounds of biological and non-biological origin that cause an increase in secondary metabolite production when applied to plants or plant-cell cultures. Many different and diverse compounds can act as elicitors, depending upon their nature of origin and their mode of action with cellular metabolism.

Examples of suitable elicitor agents include biotic elicitors such as: Botrytis cinerea Phytophthora megasperma, Pinellas stripticum, Oligosporas sp., Pythium mamiallatum, Pythium sylvaticum, Verticillium dahliae, Verticillium sp., Penicillium minioluteum, Phytophthora lateralis, Cytospora cincta, Cytospora leucostoma, Alternaria brassicicola, Alternaria solani, Alternaria cucumerina, Botrytis squamosa, Cochliobolus heterostrophus, Colletotrichum trifolii, Colletotrichum orbiculum, Colletotrichum graminicola, Colletotrichum gloeosporioides, Cylindrocladium floridanum, Fusarium crookwellense, Fusarium heterosporium, Fusarium oxysporam f. sp. conglutinans, Fusarium oxysporam f. sp. lycopersici, Fusarium oxysporam f. sp. pisi, Gibberella zeae, Gaeumaimomyces graminis var. tritici, Geotrichum sp., Leptosphaeria torrae, Nectria haematococca MPVI, Mycosphaerella pinodes, Ophiostoma ulmi, Phoma lingam, Phoma pinodella, Phytophthora infestans, Pythium aristosporum, Pythium graminicola, Pythium ultimum, Rhizoctonia solani, Sclerotinia sp., S. nodoram D-45, Trametes versicolor, Ustilago maydis, Venturia inequalis; microbial fractions or products derived from biotic elicitors such as: Chitosan, Lichenan, Glucomannan, Pleuran, Glucan, Carboxymethylglucan, Hydroxymethylglucan, Sulfoethylglucan, Mannan, Xylan, Mannobiose, Mannotriose, Mannopentaose, Mannotetraose, Cellulysin, Multifect XL, Multifect CL, Resinase, Pulpxyme, SP431, Pectinol, Rapidase, Klerzyme, Chitinase; or abiotic elicitors such as: Arachidonic acid, Elaidic acid, Cyclic AMP, Dibutyrl Cyclic AMP, Methyl Jasmone, Cis-Jasmone, Jasmonic acid, /3-glucan, Miconazol, Ferulic acid, AMO-1618, Triton X-100, Benzoic acid, Salicylic acid, Propyl gallate, Sesamol, Chlorocholine chloride, 3,4-dichlorophenoxy triethyl-, (amine), Chloroethylphosphonic acid, Diethyldithiocarbamic acid, Nordihydroguairetic acid, Dithiothreitol, Sodium metabisulfite, Potassium metabisulfite, d-amino-DL-Phenylalanine, Vanadyl sulfate, Uniconazol, Paclobutrazol, Spermine, Spermidine, Putrescine, Cadavarine, Protamine Sulfate, SKF-7997, MER 29, Ancymidol, Triadimefon, Phosphon D, Thiourea, Dextran Sulfate, Hydroquinone, Chitosan glutamate, Fenpropemorph, Prochloraz, Naptifine, EDU, HTA, MPTA, Glutathione, EGTA, Gibberellins, Abscisic Acid, 1,3-Diphenyl urea, Diazolidenyl urea, Phloroglucinol, Sodium alginate, Carrageenan, Aluminium chloride, Ethylene, Acetylsalicylic acid, Sodium chloride, Acetic acid.

It is to be understood that the elicitors mentioned above have been mentioned by way of example only, and are not intended to be limiting the scope of the invention. Howev,er particularly preferred elicitors include salicylic acid and chitosan. The at least one elicitor may comprise chitosan and/or salicylic acid to increase accumulation of benzyl isothiocyanate or any other isothiocyanate. Preferably, the at least one elicitor may comprise both chitosan and salicylic acid.

In an embodiment of the present invention, the elicitor(s) may be provided in, e.g. added to, the suspension culture in a concentration of from about 0.01 µM to about 1 M, preferably in a concentration from about 1 µM to about 500 mM, more preferably in a concentration of between about 10 µM to about 200 mM and most preferably in a concentration of between about 50 µM and about 50 mM.

Preferably, the elicitor(s) may be added to the suspension culture at a time from the inoculation time to any time during the culture duration, preferably at a time from the early exponential growth phase to the stationary phase, depending on the natures of the metabolites and the cell line of particular plant species. The at least one elicitor may be added to the suspension culture at a time from an early exponential growth phase to a stationary phase of the culture.

The at least one elicitor may be added at a plurality of times to the suspension culture, in which consecutive times of the plurality of times are separated by a period in the range of about six hours to about a month, preferably in a range between about twelve hours to about two weeks. For example, a second or multiple additions of the elicitor(s) into the suspension culture may be performed, e.g. conducted between about six hours to about a month after the previous elicitation, more preferably between about twelve hours to about two weeks after the previous elicitation, and most preferably between about twelve hours to about seven days after the previous elicitation. For example, it may be advantageous to add and/or replenish elicitors on a continuous and/or periodic basis. For example, a second or subsequent addition of the elicitor(s) into the suspension culture may be made at a time from about six hours to about a month in duration after the previous elicitation, more preferably at a time from about twelve hours to about two weeks in duration after the previous elicitation, and most preferably at a time from about 15 hours to about 5 days in duration after the previous elicitation. It may also be appropriate to initially add an elicitor(s), e.g. to the liquid medium contained at that time the cells but not yet any such elicitor, a predetermined time after the suspension culture has been established, for example a one to several hours later or 1 , 2, 4 or 6 days after suspension culture cultivation has commenced.

The liquid medium may comprise, e.g. alternatively or in addition to at least one elicitor, at least one adsorbent. For example, adsorbent may be included in, or added to, the liquid medium, e.g. to the suspension culture, in an amount of between about 1 g/L and about 500 g/L, preferably in an amount between about 20 g/L and about 300 g/L, more preferably in an amount of between about 50 g/L and 200 g/L.

For example, the adsorbent(s) may be added to the suspension culture between the inoculation to any time during the culture duration, preferably between the inoculation to the end of the exponential growth phase. The adsorbent(s) may be added to the liquid medium, e.g. to the suspension culture, in conjunction with one or a combination of elicitor agents at the same time or a different time during the cultivation, depending on the natures of the metabolites and the cell line of particular plant species.

The adsorbent(s) may comprise a macroporous non-ionic cross-linked polymeric material.

The adsorbent(s) may comprise one or more of, or may be one or more of, the following commercially available products: Amberlite XAD7, Amberlite XAD2, Amberlite XAD7HP, Amberlite XAD4, Amberlite XAD16, Amberlite XAD1600, AMBERLITE. AMBERLITE FP, Purasorb AP-250, Purasorb AP-400; Dowex L493, Dowex V493, Dowex L323, Diaion HP20, Diaion HP21, SEPABEADS SP207, SEPABEADS SP70, SEPABEADS SP700, SEPABEADS SP825, SEPABEADS SP850, Diaion HP2MG; SERDOLIT PAD I, SERDOLIT PAD II, SERDOLIT PAD III, SERDOLIT PAD IV, RP-8 (Merck), Charcoal, activated charcoal, Supelpak-2, Supelpak-2B, Supelite DAX-8, Duolite XAD761, Dowex, Optipore L493, Poly(styrene-co-divinylbenzene), AMBERSORB 572, AMBERSORB 348F, Dimethylaminomethyl-polystyrene, Poly(4- ethylstyrene-co-divinylbenzene), Florisil, Ferric hydroxide oxide, Sepiolite, Mimetic Green 1 Ligand Affinity Adsorbent, Mimetic Yellow 2 Ligand Affinity Adsorbent, Mimetic Red 2 Ligand Affinity Adsorbent, Mimetic Orange 2 Ligand Affinity Adsorbent, Mimetic Blue 1 Ligand Affinity Adsorbent, Mimetic Blue SA Ligand Affinity Adsorbent, Mimetic Blue 2 Ligand Affinity Adsorbent, Mimetic Orange 3 Ligand Affinity Adsorbent, Mimetic Red 3 Ligand Affinity Adsorbent , Mimetic Blue AP Ligand Affinity Adsorbent, Mimetic Orange 1 Ligand Affinity Adsorbent, Mimetic Yellow 1 Ligand Affinity Adsorbent, Tenax TA, AMBERCHROM , AMBERJET, AMBERLYST , DUOLITE , MAC HP , Acrylic anion resins, XAD polymeric adsorbents, Phenol- formaldehyde resin , Nuclear grade resins.

Preferably, in an embodiment of the present invention, the adsorbent(s) may comprise an aliphatic adsorbent, such as HP2MG and XAD-7, which are particularly preferred.

The adsorbent(s) may be or may comprise an immiscible liquid phase adsorbent. Examples of immiscible liquid phase adsorbents include, but are not limited to, dimethyl siloxane polymer (Silicone antifoam A), polymethoxy silanes (also known as silicone oils), long chain or branched (eg. having at least 8 and preferably having 12 to 20 carbon atoms) alkane adsorbents such as hexadecane and glycol or polyol adsorbents such as Myglyol.

The liquid culture in the shake flask(s) may be exposed to air and preferably shaken and otherwise gently moved to introduce air into the medium, or air may be introduced through tubing into the culture vessel. The culture may be maintained under appropriate growth conditions at a temperature preferably between about 15°C to 27°C, more preferably at 25°C. The pH may be from between about 3 to about 7.5 and preferably between about 5.7 to 5.8. The culture may be grown under light conditions ranging from total darkness to total light (narrow band and/or broad spectrum) for various periods of time. The culture conditions may be varied depending upon the plant cell species being cultured and upon the secondary metabolite or metabolites of interest.

Oxygen can dramatically affect the rate of secondary biosynthesis. A high saturation constant for an oxygen-requiring step on a secondary biosythetic pathway may require cells to be subjected to high oxygen levels in the shake flasks and/or the bioreactor. CO₂ supplementation may also be used to maintain high growth rates. Ethylene, a plant hormone, plays pleiotropic roles in all aspects of plant growth and development, including secondary metabolism, and may therefore also be added to the shake flasks and/or the bioreactor in a following phase of the method in accordance with embodiments.

In the shake flasks, and/or in the following bioreactor, biosynthesis of secondary metabolites may also be stimulated by medium exchange, perhaps due to removal of the product to thereby prevent feedback inhibition and product degradation. The periodic removal of spent medium incorporates production yield advantages, and additionally, may serve to depress secondary biosynthesis by removing other, non-desired secondary metabolites which exhibit an inhibitory activity. The replenishment of fresh medium to cells undergoing active biosynthesis may also enhance production by providing essential nutrients that have been depleted. It is to be recognised that the amount of medium exchange, the frequency of exchange, and the composition of the medium being replenished may be varied, depending upon the specific circumstances. For example, medium may be exchanged on a continuous or periodic basis, such as for example hourly, daily, on alternate days or weekly.

The method comprises transferring cells from the suspension culture to a further suspension culture, e.g. containing a fresh liquid medium, in a bioreactor, e.g. a large wave bioreactor, containing the at least one elicitor and/or adsorbent and/or at least one further elicitor and/or adsorbent. The liquid medium in the bioreactor may be of similar composition as used in the suspension culture in the shake flasks.

The operating mode for a plant cell culture process refers to the way that nutrients, cells and products are added or removed with respect to time. When all the nutrients are supplied initially, and the culture contents comprising cells and product are harvested at the end of the culture period, the operating mode is termed a "one-stage batch process". When a batch process is divided into two sequential phases, a growth and production phase, with the medium being exchanged in between the two phases, the operating mode is termed a "two-stage batch process".

A "FED-batch" operation, particular medium additives and nutrients are supplied either periodically or continuously through the course of a one-stage or a two-stage batch culture. When a substantial portion, but not all, of the contents of a batch culture is harvested, with additional fresh medium for continued cell growth and production, the process resembles a "repeated draw and fill" operation, and is termed a "semi-continuous process".

When fresh medium is continuously supplied, and effluent medium is continuously removed, the process is termed "continuous". If cells are retained within the reactor, the process is termed a "perfusion mode". If cells are continuously removed with the effluent medium, the continuous process is termed a "chemostat".

It is to be recognised that these various modes of process operation are compatible with the secondary metabolite production methods, e.g. particularly individually to the step of callus initiation, the step of suspension culture in shake flasks and/or the step of further suspension culture in the bioreactor, described herein.

The method comprises extracting and/or purifying and/or analysing of at least one isothiocyanate from cells in the suspension culture. This extracting and/or purifying may comprise isolating the at least one isothiocyanate as a secondary metabolite of the cells from the liquid medium comprising the cells by solvent extraction. Preferably, the recovery of the at least one isothiocyanate as secondary metabolite from the suspension culture is performed by isolating the secondary metabolite from the cells, the adsorbent and the nutrient medium utilising solvent extraction with a suitable solvent.

The method may further comprise adding the obtained isothiocyanate(s) to a pharmaceutical product, a food or drink product, supplement or additive, a skin or hair product, or an agricultural product.

The media, e.g. the callus induction medium and/or the liquid medium, described hereinabove may achieve a good total secondary metabolite formation, but may also advantageously direct cellular biosynthesis towards secondary metabolite production. The media may promote prolonged cell viability and biosynthesis, and in addition, may cause significant levels of product to be secreted into the extracellular medium. These characteristics can be particularly important in the operation of an efficient commercial scale process for secondary metabolite production.

In a further aspect, the present invention relates to the use of a method in accordance with the first aspect of the present invention for manufacturing a pharmaceutical product, a food or drink product, a supplement, an additive, a skin care product, a hair care product, or an agricultural product.

Thus, a use in accordance with embodiments may comprise providing a pharmaceutical composition comprising the isothiocyanate(s) produced by a method in accordance with embodiments of the present invention and a pharmaceutically acceptable excipient. For example, the pharmaceutical composition may be, without limitation, an anti-cancer, antibiotic, antifungal, antihistamine, anti- hypertension, anti-protzoal, antifilarial, anti-malarial, anti-schistosomal, anti-ulcer, anti-coagulant, anti-anxiety, anti-inflammation, antiseptic, nematocidal, antiviral, vasodilators, and protective/prophylactic. The composition may be suitable, without limitation, for being administered orally, nasally, parenterally, intrasystemically, intraperitoneally, topically (as by drops of transdermal patch), bucally, or as an oral or nasal spray. The pharmaceutical composition may be suitable for human or veterinary applications. The pharmaceutical composition, or the skin care product, may be suitable for treating or preventing skin cancer in a mammal, e.g. a human. The pharmaceutical composition may be suitable for treating or preventing allergic response, arterial occlusion, Alzheimer's Disease, cancer, hypo- cholesterolemia, chronic gastritis, hypertension, joint inflammation (arthritis), macular degeneration, stomach ulcers and gastritis, stroke and upper airway diseases in a mammal, e.g. a human.

A use in accordance with embodiments may comprise providing a food or drink product, supplement or additive comprising the isothiocyanate(s) produced by a method in accordance with embodiments of the present invention, in which the food or drink product or supplement may be, without limitation, a juice, smoothie, shake, tea, soup, sauce, salad, granolas, cereals, bread, other baked good, fried good, pill, spray and/or other ingestible product. The food or drink product, supplement or additive may be suitable for human or veterinary applications.

A use in accordance with embodiments may comprise providing a skin or hair care product comprising the isothiocyanate(s) produced by a method in accordance with embodiments of the present invention. The skin or hair care product may be, without limitation, a hair detergents such as shampoo, rinse, rinse-in- shampoo, conditioning shampoo, and the like; a hair cosmetic, including hair lotion, hair conditioner, hair treatment, hair cream, hair spray, hair liquid, hair wax, hair water, hair-styling preparation, perming liquid, hair color, acidic hair color, hair manicure, etc., or a skin cosmetic such as skin lotion, milky lotion, face wash, makeup remover, cleansing lotion, emollient lotion, nourishing cream, emollient cream, massage cream, cleansing cream, body shampoo, hand soap, bar soap, shaving cream, sunburn cosmetics, deodorant gel, deodorant powder, deodorant lotion, deodorant spray, anti-perspirant gel, anti-perspirant powder, anti-perspirant lotion, anti-perspirant spray, combination deodorant & anti-perspirant gel, combination deodorant/anti-perspirant powder, combination deodorant/anti-perspirant lotion, combination deodorant/anti-perspirant spray, makeup removing gel, moisture gel, moisture essence, UV -preventing essence, shaving foam, face powder, foundation, lipstick, cheek rouge, eyeliner, eye shadow, eyebrow pencil, bathing preparation, etc.; mouth detergent such as toothpaste; or other hair and skin products. The skin or hair product may be suitable for human or veterinary applications.

Another use in accordance with embodiments of the present invention may comprise providing an agricultural product comprising the isothiocyanate(s) produced by a method in accordance with embodiments of the present invention. The agricultural product may be, without limitation, an agricultural pesticide, powder, pellet, spray, fertilizer, side-dressing, in-furrow amendment, soil amendment, compost or other agricultural product.

In a use in accordance with embodiments, the isothiocyanate(s) may be provided in the pharmaceutical product, food or drink product, supplement, additive, skin care product or hair care product to induce the activity of phase 2 enzymes in a mammal, e.g. a human.

In a use in accordance with embodiments, the isothiocyanate(s) may be provided in the agricultural product to induce the activity of phase 2 enzymes in a plant.

Hereinbelow, examples and experimental results are presented for illustrating aspects and applications of embodiments of the present invention. It shall be understood that such examples and/or experimental results are merely illustrative and not intended as limiting the present invention. However, a process described in the examples hereinbelow may be considered as a preferred embodiment of the present invention. A similar approach, with obvious changes within the capabilities of the skilled person may be carried out to produce different isothiocyanates, or isothiocyanates in different preferred ratios with respect to each other, and/or from different core brassicales plant species, and therefore considered as equally within the scope of the present invention.

### SEED PREPARATION & GERMINATION

Conditions for in vitro mass production of cabbage callus were determined to identify the most responsive callus induction in Cabbage. For this purpose, a variety of cabbage, comprising NON-GMO Impala F1 Cabbage (Brassica oleracea L. var. Capitata); was selected. The F1 hybrid seeds of said cultivar were purchased from Technisem, Longué-Jumelles, France. The seeds were left under running tap water for 60 minutes. The seeds were surface sterilized using 70% Ethanol and 0.1% Mercuric chloride solution (Sigma-Aldrich, Belgium) in distilled water. The seeds were soaked in this solution for 5 minutes and washed thrice with autoclaved distilled water under aseptic condition. This was done due to remove the seed coating and the dust that surround the seeds. After third washing seeds were kept in autoclaved distilled water for five minutes and then dried on autoclaved pieces of Whatman cellulose filter paper (Sigma-Aldrich, Belgium) in laminar airflow cabinet.

For germination, seeds were placed on moist paper in disposable Petri plates; plates were sealed with parafilm (Sigma-Aldrich, Belgium) and incubated in dark at 20 ± 2°C. The hypocotyls of 5-7 days old seedlings were used as explant for callogenesis. The callus induction medium contained 4.43g/l Murashige and Skoog (MS) vitamin solution (M3900, Sigma-Aldrich, Belgium. solution contains (mg/ml): 2.0 glycine, 100.0 myo-inositol, 0.50 nicotinic acid, 0.50 pyridoxine hydrochloride, 0.10 thiamine hydrochloride), 3.0% analytical grade sucrose (Amsbio, UK), 0.6% gelling source (Phytagel; Sigma-Aldrich, Belgium). To study the ideal ratios for inducing callogenesis and secondary metabolites, filter sterilized hormonal supplementations; Auxin; 2,4-d (2,4-dichlorophenoxyacetic acid; Sigma-Aldrich, Belgium), Cytokinin; 6-BAP (6-Benzylaminopurine; Sigma-Aldrich, Belgium), and amino acid precursor were used as various concentrations. The pH of medium was adjusted to 5.5 and 5.7 with 0.1 NaOH and 0.1 N HCI (Sigma-Aldrich, Belgium) prior to gelling. Media (50 - 55ml volumes) were dispensed into large sterile jars. Jars were sealed with autoclavable polypropylene caps and autoclaved for 15 minutes at 121°C under 1.1 kg/cm2 pressure. The media were then left to cool and stored at room temperature till used.

### CALLUS INDUCTION

All cultures were incubated in an air conditioned incubation room. The callus cultures were kept at 25±2°C in complete darkness to avoid browning of tissue due to phenolic compounds.

To study the callus induction response of different cultivars, fifteen different callus induction media were examined. Hypocotyls explants of NON-GMO Impala F1 Cabbage (Brassica oleracea L. var. Capitata) were simultaneously cultured on all callus induction media given in various concentrations of 2,4-D, and 6-BAP. Generally within two weeks of first inoculums, the pale yellow color of explants tissue turned greenish along with increase in length approximately up to double of its original length. Then with passing days whole of the explants tissue turned into a fresh yellow colored mass of embryogenic callus. Tabulated data helped to infer that CM1, CM3, CM6 and CM9 media were most effective for callus induction as all of the explant tissues cultured on these media produced very viable and embryogenic calli (100% callus induction). Tabulated observations expressed the fact that high concentration of 2,4-D in medium favors the repeated cell division and formation of callus and found better if used in ratio of 2:1 (at least) with auxin, respectively.

Callus cultures were sub-cultured into fresh culture media after 4 weeks to increase the total weight of the callus culture. Before transferring to an optimized liquid culture media using a single-use bioreactor bag, the percentage of callus induction (%), mean fresh and dry weights of callus (mg/explant), and both color and texture of callus were recorded. The detailed composition of the optimized medium for cell suspension culture of Benzyl Isothiocyanate production from NON-GMO Impala F1 Cabbage (Brassica Oleracea L. var. Capitata) is shown in the table hereinbelow. The original MS medium contained NH₄NO₃ and was replaced by NaNO3+ inclusion of vitamin B spectrum.

| Micro elements | mg/l | µM |
|---|---|---|
| CoCl₂.6H₂O | 0.025 | 0.11 |
| CuSO₄.5H₂O | 0.025 | 0.10 |
| FeNaEDTA | 36.70 | 100.00 |
| H₃BO₃ | 6.20 | 100.27 |
| Kl | 0.83 | 5.00 |
| MnSO₄.H₂O | 16.90 | 100.00 |
| Na₂MoO₄.2H₂O | 0.25 | 1.03 |
| ZnSO₄.7H₂O | 8.60 | 29.91 |

| Macro elements | mg/l | mM |
|---|---|---|
| CaCl₂ | 332.02 | 2.99 |
| KH₂PO₄ | 170.00 | 1.25 |
| KNO₃ | 1900.00 | 18.79 |
| MgSO₄ | 180.54 | 1.50 |
| NaNO₃ | 1751.00 | 20.61 |

| Vitamins | mg/l | µM |
|---|---|---|
| myo-inositol | 100.00 | 554.94 |
| nicotinic acid | 1.00 | 8.12 |
| pyridoxine HCl | 1.00 | 4.86 |
| thiamine HCl | 10.00 | 29.65 |

### ELICITATION

10g of calli (fresh weight/vessel) was transferred to the same medium composition in addition to different concentrations of chitosan (200, 400 and 600mg/L) and salicylic acid (10, 20 and 40mg/L) as biotic elicitors, in addition to the control treatment without elicitors. After calli was collected, the fresh and dry weights were determined. Dry weight was determined after drying the calli at 45°C in an oven until a constant weight was obtained. Benzyl Isothiocyanate concentration was also recorded with each treatment. After four weeks of culture, data was documented.

### ESTABLISHMENT OF SUSPENSION CULTURES - ERLENMEYER FLASKS

Suspension cultures were established from 6 g fresh friable callus in 200 ml Erlenmeyer flasks (Saint Gobain Performance Plastics Chemware PFA Graduated Erlenmyer Flask) containing 60 ml of the liquid optimized MS medium that containing 1.0 mg/L of each of 2,4-D and Kin, in addition to 200 mg/L chitosan. The pH of the medium was adjusted to 5.7-5.8 before autoclaving as mentioned previously. The flasks were closed with sterile non-absorbent cotton plugs and two layers of aluminum foil and incubated on a rotary shaker (IKA Laboratory Equipment, Belgium) with speed of 100 rpm at 25±2°C under a 16-hour photoperiod in the growth room (Conviron, Germany). Cultured cells were sampled at each growth stage up to the 28th day of incubation in 7-day intervals and Benzyl Isothiocyanate concentration was determined.

### ESTABLISHMENT OF SUSPENSION CULTURES - BIOREACTOR EXPERIMENT

A single-use ReadyToProcess WAVE Cellbag bioreactor (GE Life Sciences, Belgium) was used in the present study; consisting of a 5L disposable cellbag. An amount of 40g of Impala F1 Cabbage (Brassica oleracea L. var. Capitata) cells were cultivated into the culture cellbag containing 4L (working volume) of the optimized medium (Murashige and Skoog medium, supplemented with 1mg/L of each of 2,4-D and Kin, in addition to 200mg/L chitosan). Rocking angle was kept at 6°, rocking rates were kept at 20 rocks/min and temperature was kept at 25°C. pH was maintained at 5.7-5.8.

Samples were collected in 7-day intervals up to the 30th day for the quantitative analysis of Benzyl Isothiocyanate.

### ANALYSIS OF BENZYL ISOTHIOCYANATE CONTENT

To determine Benzyl Isothiocyanate contect, high performance liquid chromatography (HPLC) was performed. The extraction of Benzyl Isothiocyanate was carried out from calli and cells from Erlenmeyer flask culture and single-use ReadyToProcess WAVE Cellbag bioreactor experiments. Samples were homogenized using a glass mortar and pestle (MilliporeSigma, Belgium) containing 70:30 (v:v) MeOH:sterilized water. Extracts were then transferred to glass stoppered Erlenmeyer flasks (DWK Life Sciences) and conditioned in a thermostatic bath (Fisherscientific, Belgium) under constant agitation. For 30 minutes the extraction was carried out at 70°C and then filtered on Invitrolon PVDF membranes (Polyvinylidene Fluoride) (0.45µm, ThermoFisher Scientific, Belgium) prior to HPLC injection.

Methanolic extracts of the samples were analyzed by HPLC for Benzyl Isothiocyanate concentration using an external standard. HPLC system (Ultimate 3000 Automated System, ThermoFisher Scientific, Belgium) was coupled with UV-Vis detector (UltiMate 3000 Multiple Wavelength Detector, ThermoFisher Scientific, Belgium) and vacuum degasser (UltiMate 3000 SRD-3400 HPLC Degasser, ThermoFisher Scientific, Belgium).

Sample injections of 20µl were made by an autosampler (UltiMate 3000 WPS-3000 Autosampler, ThermoFisher Scientific, Belgium); the chromatographic separations were performed on C18 Reversed Phase LC columns (Accucore C18 LC Columns, ThermoFisher Scientific, Belgium). Column temperature was maintained at 30°C and detection wavelenght was 253nm. Optimum efficiency of separation was obtained using methanol (solvent A), and acetonitrile (solvent B) (HPLC grade, Sigma Aldrich, Belgium). The flow rate was 1ml/minute. The running time for each analysis was 60 minutes, and 10 minutes were required for column cleaning and equilibrium.

The Benzyl Isothiocyanate concentration of the injected samples was calculated as µg/g fresh weight of sample. Experiments were conducted in a completely randomized design with at least 30 replicates per treatment for the calli experiment and 4 replicates for the cell suspension culture experiments. Data were subjected to statistical analysis by Analysis of Variance (Anova) programme using Duncan's multiple range test as modified by Snedecor and Cochran. Values marked with different letters were considered to be statistically different at p<0.04.

### OBSERVED RESULTS

NON-GMO Impala F1 Cabbage (Brassica oleracea L. var. Capitata) callus cultures derived from Hypocotyls explants accumulated Benzyl Isothiocyanate as a response to salicylic acid and chitosan, which acted as biotic elicitors. These two elicitors were screened for various concentrations for high quantity of Benzyl Isothiocyanate accumulation. Both salicylic acid and chitosan showed significant increase in Benzyl Isothiocyanate accumulation over control cultures with insignificant increase in the fresh weight of calli. Calli treated with chitosan increased Benzyl Isothiocyanate accumulation compared with that of salicylic acid. Maximum accumulation of Benzyl Isothiocyanate (6.425µg/g fresh weight) was recorded after the treatment with chitosan at the concentration of 200mg/L, which achieved about 5.1-fold increase in the Benzyl Isothiocyanate production over the control treatment (1.248 µg/g fresh weight), and also achieved the highest fresh and dry weight of calli. Despite this treatment was the optimum, Benzyl Isothiocyanate production decreased above this concentration level of chitosan (400 and 600mg/L) in the medium. The callus treated with different concentrations of salicylic acid (10 - 40mg/L) showed maximum Benzyl Isothiocyanate accumulation (2.174 µg/g fresh weight) at 10mg/L, which slightly raised the yield of Benzyl Isothiocyanate over the control. It recorded an increase of 1.7 times over the control treatment. The increase in salicylic acid elicitor concentration led to a decline in the Benzyl Isothiocyanate accumulation. Results are presented in the table hereinbelow.

| Elicitor concentration (mg/L) | Fresh weight (g) | Dry weight (g) | Concentration of benzyl isothiocyanate (µg/g fresh weight) |
|---|---|---|---|
| Control | | | |
| 0 | 5.061 | 4.637 | 1.248 |

| Chitosan | | | |
|---|---|---|---|
| 200 | 6.306 | 5.811 | 6.425 |
| 400 | 5.718 | 5.041 | 3.914 |
| 600 | 4.963 | 4.871 | 3.498 |

| Salicylic acid | | | |
|---|---|---|---|
| 10 | 5.985 | 4.807 | 2.174 |
| 20 | 4.981 | 4.424 | 2.039 |
| 40 | 5.509 | 5.107 | 1.956 |

The best dose of the elicitors with maximum accumulation of Benzyl Isothiocyanate was used in further experiments (200mg/L chitosan). It was observed that Benzyl Isothiocyanate production is highly affected by the addition of biotic elicitors. Therefore, elicitation offers an attractive strategy for increasing the secondary metabolite in vitro production. The experiments showed that both chitosan and salicylic acid increased accumulation of Benzyl Isothiocyanates in callus culture of NON-GMO Impala F1 Cabbage (Brassica oleracea L. var. Capitata) over the control.

The stimulatory effect of chitosan and salicylic acid might be due to the stimulation of biosynthetic enzymes. Chitosan is the main structural component of the cell wall of plant pathogen fungi, which mimics its effects and activates the biosynthesis of defense-related secondary metabolites in plants. Also, salicylic acid has an important role in the defense against attacks by microbes and herbivores, and against abiotic stresses.

It was observed that chitosan better enhanced the production of Benzyl Isothiocyanate than salicylic acid. Another significant observed effect of the elicitors was that the accumulation of Benzyl Isothiocyanate is elicitor dose dependent. The increase in Benzyl Isothiocyanate accumulation with elicitor application was observed and the increase in elicitor concentration over the optimum concentration led to decrease in the Benzyl Isothiocyanate content in callus cultures.

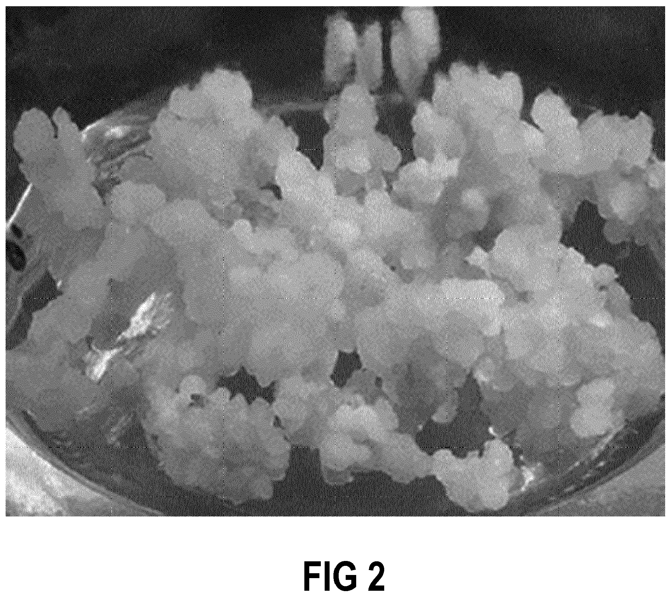

FIG 2 shows a callus of NON-GMO Impala F1 Cabbage (Brassica Oleracea L. var. Capitata) as obtained in the present experiment.

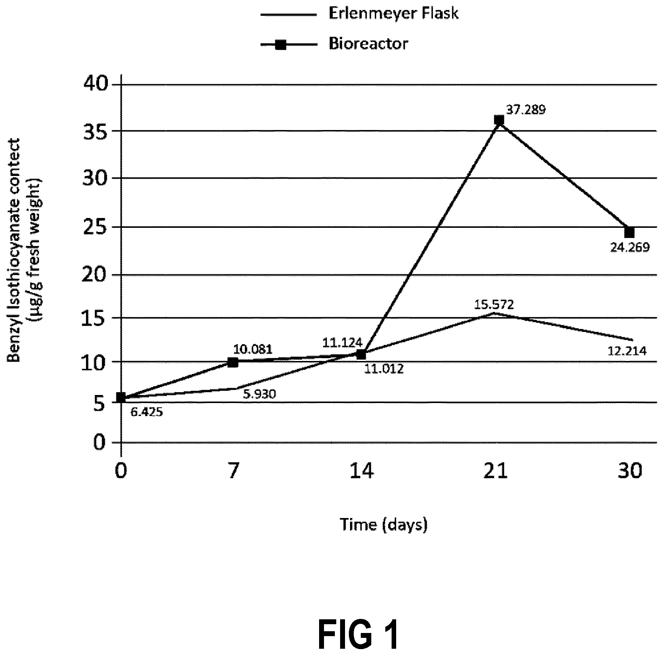

Cell suspension cultures offer an alternative way to traditional agriculture for industrial production of various valuable phytochemicals. The liquid culture has many advantages compared to solid one. The contacting surface area of cells and media is much larger, which permit cells to utilize the well-mixed nutrients much easier. Also, harmful compounds that may be formed can be effectively diluted, which prevents the inhibition of cell growth. FIG. 1 shows the effect of elicitation with the optimum concentration of chitosan (200 mg/L) on Benzyl Isothiocyanate accumulation in Erlenmeyer-flask culture and single-use ReadyToProcess WAVE Cellbag bioreactor during different durations of incubation time. Successful scale-up of Benzyl Isothiocyanate accumulation was achieved from cell suspension cultures using both methods.

Its accumulation varied from 7 to 30 days of incubation and increased with increasing in the duration of incubation time. This result indicates that the duration of cell cultured with the elicitor may be important for a maximum Benzyl Isothiocyanate accumulation. The amount of metabolite production may vary with duration time of incubation with elicitors. The optimum incubation time for Benzyl Isothiocyanate accumulation with chitosan was found to be 21 days for both shake-flask culture and bioreactor.

Comparing Benzyl Isothiocyanate accumulation in NON-GMO Impala F1 Cabbage (Brassica oleracea L. var. Capitata) cell cultures between Erlenmeyer-flask and the single-use ReadyToProcess WAVE Cellbag bioreactor system is shown in FIG. 1.

In general, Benzyl Isothiocyanate yield was higher in the bioreactor than that using Erlenmeyer-flask culture during all the studied durations. Using the bioreactor, Benzyl Isothiocyanate content was 10.078 and 11.98µg/g fresh weight after 7 and 14 days of incubation, respectively.

Benzyl Isothiocyanate content increased significantly after 21 days, reaching the maximum value of 37.289 µg/g fresh weight, which achieved about 5.8-fold increase, then it declined (reaching 24.269 µg/g fresh weight of only 3.7-fold increase). The Benzyl Isothiocyanate content was higher in the bioreactor by about 1.7, 1.3, 2.5 and 2 times than the shake-flask culture after 7, 14, 21 and 30 days, respectively. Only after 14 days they were more or less the same. Both gave the maximum Benzyl Isothiocyanate accumulation after 21 days and declined after 30 days. The decreased Benzyl Isothiocyanate accumulation could be due to arrest of cell growth, degradation or conversion of Benzyl Isothiocyanate to some other chemical forms or due to change in medium nutrient composition.

The different performance between shake-flask culture and the bioreactor could be due to the difference in hydrodynamic conditions and gas compositions between the two systems. The large surface area for the air-medium interface in the Erlenmeyer flask and the single-use ReadyToProcess WAVE Cellbag bioreactor (larger container) provide an adequate amount of oxygen and nutrient mass transfer. In general, an application of a bioreactor may be considered a prerequisite for industrialization of plant cell culture. They can provide well mixed media and sterile air.

## Claims

1. A method of producing isothiocyanates, the method comprising:
- forming, in a semi-solid or solid callus induction medium, compact callus aggregates from cells obtained from explant material of a plant of a Brassicales plant species;
- transferring cells from the callus aggregates to a suspension culture in a liquid medium in a shake flask, the liquid medium containing at least one elicitor and/or adsorbent,
- transferring cells from the suspension culture to a further suspension culture in a bioreactor containing the at least one elicitor and/or adsorbent, and
- extracting and/or purifying of at least one isothiocyanate from cells obtained from the bioreactor.

2. The method of claim 1, wherein said extracting and/or purifying comprises isolating the at least one isothiocyanate as a secondary metabolite of the cells from the bioreactor by solvent extraction.

3. The method of claim 1 of claim 2, wherein said cells obtained from explant material are derived from leaves, fruit, shoots, buds, flowers, bark, roots, branches, stems, seeds, cones, needles or cambium tissue of the plant.

4. The method of any of the previous claims, wherein said cells obtained from explant material are obtained from hypocotyl explant material and/or wherein said cells are derived from meristematic plant tissue.

5. The method of any of the previous claims, wherein said cells obtained from explant material are obtained from explant material of a plant species that produces the secondary metabolite benzyl isothiocyanate and/or other isothiocyanates.

6. The method of any of the previous claims, wherein said cells obtained from explant material are Brassica oleracea L plant cells.

7. The method of any of the previous claims, wherein said callus induction medium comprises a Murashige and Skoog medium and/or a B-5 medium,

8. The method of any of the previous claims, wherein said at least one elicitor comprises at least one abiotic elecitor and/or at least one biotic elicitor and/or at least one microbial fraction or product derived from a biotic elicitor.

9. The method of claim 8, wherein said at least one elicitor comprises chitosan and/or salicylic acid to increase accumulation of benzyl isothiocyanate or any other isothiocyanate.

10. The method of any of the previous claims, wherein said at least one adsorbent comprises an aliphatic adsorbent and/or an immisible liquid phase adsorbent.

11. The method of any of the previous claims, wherein said at least one elicitor and/or adsorbent is added to the suspension culture at a time from an early exponential growth phase to a stationary phase of the culture.

12. The method of any of the previous claims, wherein said at least one elicitor and/or adsorbent is added at a plurality of times to the suspension culture, in which said plurality of times are separated by a period in the range of about six hours to about a month.

13. The method of any of the previous claims, wherein said induction medium comprises an auxin and/or a cytokinin and/or a giberellin.

14. The method of any of the previous claims, wherein the callus aggregates and/or the suspension culture and/or the further suspension culture is repeatedly sub-cultured.

15. A use of a method in accordance with any of the previous claims for manufacturing a pharmaceutical product, a food or drink product, a supplement, an additive, a skin care product, a hair care product, or an agricultural product.
